# EUROPEAN PATENT APPLICATION

(11) **EP 1 972 277 A1**
(43) Date of publication of application: **24.09.2008**
(21) Application number: 07425161.2
(22) Date of filing: 20.03.2007
(51) Int. Cl.: A61B 6/03, A61B 6/14

(54) **Method for positioning an object to be analysed for a computed tomography scanner**

(71) Applicant: CEFLA SOCIETA' COOPERATIVA, 40026 Imola (BO) (IT)
(72) Inventor: Pasini, Alessandro, 47023 Cesena (IT); Nanni, Eros, 40023 Castel Guelfo di Bologna (IT)
(74) Representative: Jorio, Paolo

(57) **Abstract**

In a computed tomography scanner having a support (3) for supporting an object to be analysed in an area of analysis (5), and a source-detector assembly (4) for emission of a given radiation and for detection of said radiation, which rotates about the area of analysis (5) for acquiring radiographies of the object, the support (3) being mobile with respect to the source-detector assembly (4) along three axes of displacement (18-20), at least one scout-view of the object is acquired via the source-detector assembly (4) and is displayed via a monitor (14), the co-ordinates of a desired point (P) of the object are acquired from the scout-view displayed and are processed to obtain actual displacements to be imparted on the support (3) along the axes of displacement (18-20) so as to centre the object in the area of analysis (5) with respect to the desired point (P), and the support (3) is moved according to said actual displacements by actuating three actuators (21-23).

## Description

The present invention relates to a method for positioning an object to be analysed for a computed tomography scanner.

In particular, the present invention finds advantageous, but not exclusive application in computed tomography scanners used in the sector of dentistry, to which the ensuing description will make explicit reference, without this implying any loss of generality.

Computed tomography scanners used in the field of dentistry are of the type comprising a frame, on which a couch is mounted for supporting a patient lying down, and an x-ray source-detector assembly designed to rotate about an area of analysis located in a region corresponding to a headrest of the couch, on which the head of the patient is positioned, for acquiring a plurality of radiographies of a particular anatomical area of interest of the head of the patient, for example, one or both of the dental arches.

The tomography scanner further comprises a control unit connected to the source-detector assembly for controlling emission and reception of the beam in a way synchronous with rotation of the arm, and a processing unit connected to the detector for receiving, storing, and processing the radiographies acquired for different angular positions of the rotating support so as to be able to reconstruct images of the dental arches. The processing unit is equipped with a monitor for enabling display of the reconstructed images.

Once the patient has been positioned on the couch, before carrying out acquisition of the radiographies necessary for reconstruction of the images sought, a pair of scout-views of the head of the patient are normally acquired, said scout-views being displayed on the monitor for checking that the anatomical part of interest is centred in the area of analysis and hence ensuring that said anatomical part will be captured correctly during the subsequent acquisition of the radiographies.

In the case where the anatomical part of interest is not centred, the tomography scanner operator must correct the position of the patient by displacing his head manually on the headrest of the couch. This operation involves numerous attempts, after as many acquisitions of scout-views, which administer useless x-ray doses to the patient.

The aim of the present invention is to provide a method for positioning the patient on the couch of a computed tomography scanner, and a computed tomography scanner implementing said method that will enable the drawback described above to be overcome and, at the same time, are easy and inexpensive to produce.

According to the present invention, a method for positioning an object to be analysed for a computed tomography scanner, and a computed tomography scanner are provided in accordance with the annexed claims.

For a better understanding of the present invention, a preferred embodiment is now described, purely by way of nonlimiting example and with reference to the attached plates of drawings, wherein:
- Figure 1 illustrates a computed tomography scanner implementing the method for positioning an object to be analysed according to the present invention; and
- Figures 2 and 3 illustrate the method for positioning an object to be analysed according to the present invention performed starting from an example of scout-view.

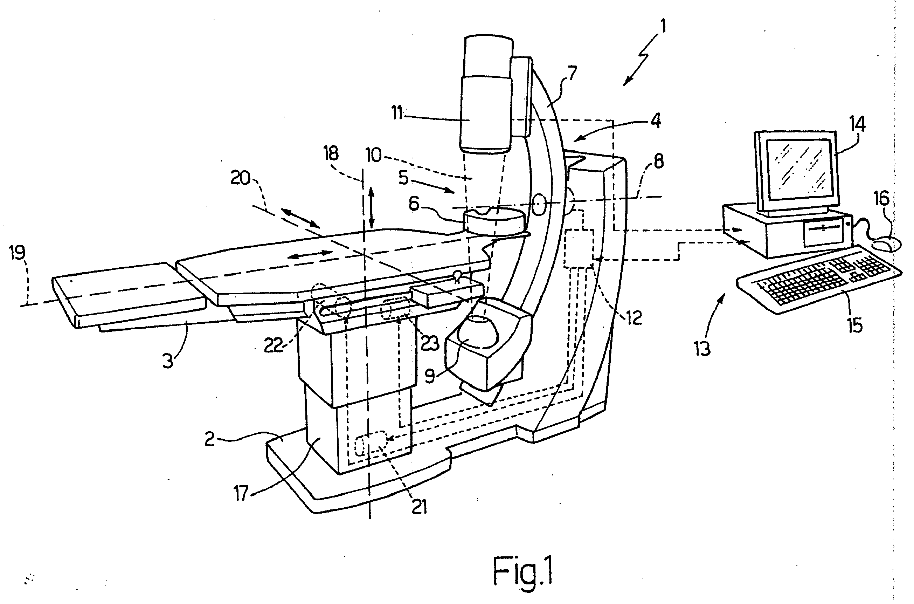

In Figure 1, designated as a whole by 1 is a computed tomography scanner of the type used in dentistry, comprising a frame 2, a couch 3 for supporting a patient (not illustrated) lying down, and an x-ray source-detector assembly 4 designed to rotate about an area of analysis 5 located in a region corresponding to a headrest 6 of the couch 3 for acquiring a plurality of radiographies of a part of the head of the patient, for example, a maxillofacial complex, such as the dental arches, the mandibular bone or else the maxillary bone of the patient, which hereinafter will be referred to as "object" for reasons of simplicity.

The source-detector assembly 4 comprises a rotating support constituted by an arm 7 mounted on the frame 2 and motor-driven so as to rotate about a substantially horizontal axis of rotation 8 traversing the region of analysis 5, an x-ray emitter 9 mounted on a first end of the arm 7 and facing in the direction of the axis 8 for emitting a conical beam 10 of x-rays towards the area of analysis 5, and an x-ray detector 11 mounted on the opposite end of the arm 7 and facing in the direction of the axis 8 for receiving the beam 10 after it has traversed the area of analysis 5 so as to be able to acquire a plurality of radiographies of the dental arches during a single rotation of the arm along a given angular path.

The tomography scanner 1 further comprises a control unit 12 connected to the source-detector assembly 4 for controlling emission and reception of the beam 10 in a way synchronous with rotation of the arm 7, and a processing unit 13 connected to the detector 11 for receiving, storing, and processing the radiographies acquired so as to reconstruct images of the object and to the control unit 12 for activating the source-detector assembly 4 on the basis of commands imparted by an operator or of instructions with which the processing unit 13 itself is programmed. The processing unit 13 is constituted, for example, by a personal computer provided with a monitor 14 for displaying the reconstructed images, and a keyboard 15 and a mouse 16 for acquiring data supplied and/or commands imparted by the operator.

According to the present invention, the frame 2 comprises a base 17, and the couch 3 is mounted on the base 17 so as to move, with respect to the frame 2 and hence with respect to the source-detector assembly 4, along a vertical axis 18, along a longitudinal axis 19 substantially parallel to the axis 8 of rotation, and along a transverse axis 20 substantially orthogonal to the vertical axis 18 and the longitudinal axis 19.

Housed in the base 17 are a first actuator 21, designed to lower and raise the couch 3 along the vertical axis 18, a second actuator 22, designed to move the couch 3 forwards and backwards along the longitudinal axis 19, and a third actuator 23, designed to move the couch 3 to the right and to the left along the transverse axis 20. The actuators 21, 22, and 23 are of a linear type and are kinematically coupled to the couch 3 via linear guides or wormscrews. In addition, the actuators 21, 22, and 23 are connected to the control unit 12 for receiving therefrom respective actuation commands that enable adjustment of the position of the couch 3 with respect to the source-detector assembly 4 along the three axes 18, 19, and 20. The control unit 12 sends said actuation commands on the basis of displacement instructions supplied by the processing unit 13.

Loaded into the processing unit 13 is a program for positioning the couch, which implements a method for positioning an object to be analysed according to the present invention and which will be described hereinafter.

First of all, the operator orders the tomography scanner 1 to acquire, via the source-detector assembly 4, and display a pair of scout-views of the object, for example, a latero-lateral view and an antero-posterior view. If the operator notes that the object is not centred as desired in the field of view that defines the scout-view, i.e., that the object is not centred as desired in the area of analysis 5, he supplies to the processing unit 13 the two-dimensional co-ordinates of a desired point on each scout-view, with respect to which point it is desired to centre the object in the area of analysis 5. The processing unit 13 processes said co-ordinates for producing displacement instructions to be sent to the control unit 12.

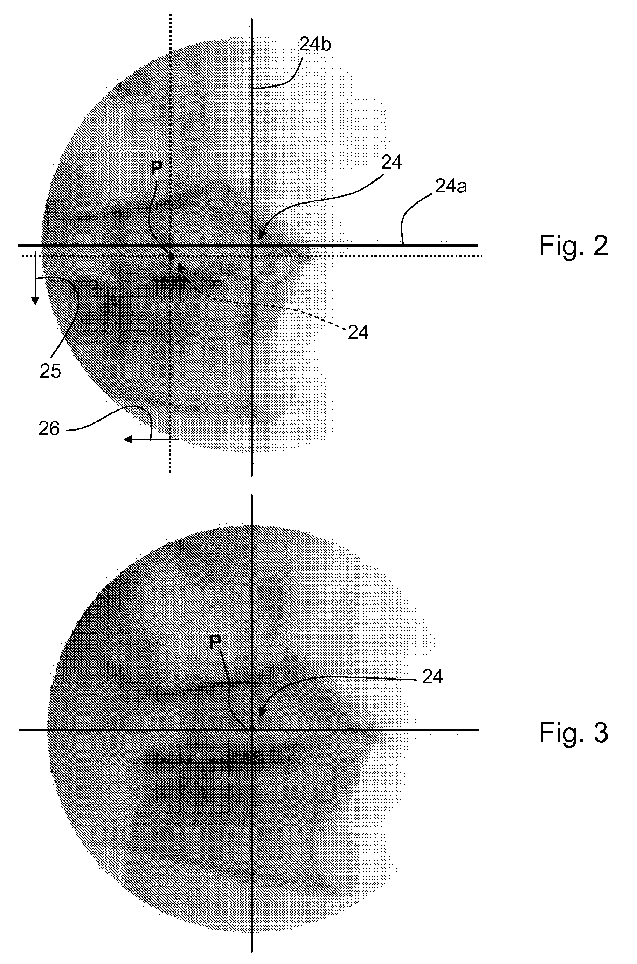

Said two-dimensional co-ordinates are acquired and processed by the processing unit 13 in the way described hereinafter, with particular reference to a latero-lateral scout-view of the dental arches of a patient illustrated in Figures 2 and 3, respectively, before and after centring.

According to a first embodiment of the present invention, the processing unit 13 generates on the scout-view a pointer 24, for example, of the type defined by the crossing-over of two axes 24a and 24b perpendicular to one another (Figure 2). The operator acts with the cursor-control keys to shift the pointer 24 on the desired point P, as shown in Figure 2 by the pointer 24 represented with a dashed line. At each pressure of a cursor-control key there is produced, along one of the directions 25 and 26 respectively perpendicular to the axes 24a and 24b, a corresponding increment of displacement of the pointer 24 equal to a given amount of pixels, said increment being stored by the processing unit 13. When the operator confirms the desired point P via the enter key of the keyboard 15, the processing unit 13 sums up the increments of displacement stored for the two directions 25 and 26 so as to determine the two-dimensional co-ordinates in pixels of the desired point P with respect to a reference system corresponding to the scout-view. Then, the processing unit 13 processes said co-ordinates as a proportion between the displacements in pixels and the mechanical displacements of the actuators 21, 22, and 23, and hence of the couch 3, and taking into account the geometry of acquisition of the scout-views so as to obtain the actual displacements to be imparted on the couch 3 along two of the axes 18, 19, and 20 for displacing the object so as to centre it in the area of analysis 5 with respect to the desired point P. The actual displacements calculated are sent to the control unit 12 with appropriate instructions.

For example, in the case where the scout-view of Figure 2 has been acquired with the face of the patient facing upwards along the vertical axis 18, the displacement of the centring cross 24 in the direction 25 is translated into a respective mechanical displacement of the couch 3 along the longitudinal axis 19 such as to move the couch 3 closer to the source-detector assembly 4, and the displacement of the centring cross 24 in the direction 26 is translated into a respective mechanical displacement of the couch 3 along the vertical axis 18 such as to raise the couch 3.

Figure 3 illustrates a latero-lateral scout-view of the same dental arches after automatic centring carried out by the tomography scanner 1.

In the case, instead, of an antero-posterior scout-view (not illustrated), acquired once again with the face of the patient facing upwards along the vertical axis 18, the two modes described above operate substantially in the same way with the difference that the processing unit 13 processes the co-ordinates in pixels acquired so as to obtain the actual displacements to be imparted on the couch 3 along the longitudinal axis 19 and the transverse axis 20.

According to a second embodiment of the present invention, the operator acts on the cursor-control keys of the keyboard 15 or else, alternatively, on the mouse 16 to displace the pointer 24 on the desired point P. The processing unit 13 reads continuously the two-dimensional co-ordinates in pixels of the pointer 24 with respect to said reference system corresponding to the scout-view during movement of the pointer 24 itself. When the operator confirms the desired point P via the enter key of the keyboard 15 or else via a key of the mouse 16, the processing unit 13 acquires the co-ordinates of the pointer 24, and hence the co-ordinates of the desired point P. Then, the processing unit 13 processes said co-ordinates so as to obtain the actual displacements to be imparted on the couch 3 in the same way as for the first embodiment described previously.

From the above description, it is clear that the method for positioning an object to be analysed according to the present invention and the tomography scanner 1 implementing said method are suitable for acquisition of radiographies for the reconstruction of images of any anatomical part of the human body or of any other type of object of biological or non-biological matter.

The advantage of the method for positioning an object to be analysed described above enables the operator to correct automatically positioning of the patient with respect to the source-detector assembly 4, and hence the head of the patient in the area of analysis 5, after observation of the scout-views, by acting directly on the processing unit 13, i.e., on the personal computer, without him having to stand up from his workstation and without him having to administer to the patient further and useless doses of radiation for acquiring other tentative scout-views.

## Claims

1. A method for positioning an object to be analysed for a computed tomography scanner comprising supporting means (3) for supporting the object in an area of analysis (5), and source and detector means (4) for emission and detection of a given radiation designed to rotate about the area of analysis (5) for acquiring radiographies of the object; said supporting means (3) being mobile with respect to the source and detector means (4) along three axes of displacement (18-20); the method comprising:
- acquiring, via said source and detector means (4), at least one scout-view of the object;
- displaying the scout-view;
- acquiring, from the scout-view displayed, the co-ordinates of a desired point (P) of the object with respect to a reference system corresponding to the scout-view itself;
- processing the co-ordinates of the desired point (P) for obtaining actual displacements to be imparted on said supporting means (3) along the axes of displacement (18-20) in such a way as to centre the object in the area of analysis (5) with respect to the desired point (P); and
- moving, via actuator means (21-23), said supporting means (3) according to said actual displacements.

2. The method according to Claim 1, in which processing of the co-ordinates of the desired point (P) is carried out according to the geometry of acquisition of the scout-views.

3. The method according to Claim 1 or Claim 2, in which said scout-view is processed for being displayed in digital format; processing of the co-ordinates of the desired point (P) is carried out as a proportion between the displacements in pixels and the mechanical displacements of said supporting means.

4. The method according to any one of Claims 1 to 3, in which acquiring, from the scout-view displayed, the co-ordinates of a desired point (P) of the object comprises:
- generating a pointer (24) on the scout-view displayed;
- positioning the pointer (24) on the desired point (P); and
- acquiring the co-ordinates of the pointer (24).

5. The method according to any one of Claims 1 to 3, in which acquiring, from the scout-view displayed, the co-ordinates of a desired point (P) of the object comprises:
- generating a pointer (24) on the scout-view displayed;
- moving the pointer (24) until it is positioned on the desired point (P);
- storing increments of displacement of the pointer (24) during the movement of the pointer (24) itself;
- determining the co-ordinates of the desired point (P) by processing said increments of displacement.

6. The method according to any one of Claims 1 to 5, in which said axes of displacement (18-20) are orthogonal to one another.

7. A computed tomography scanner comprising: supporting means (3) for supporting an object to be analysed in an area of analysis (5); source and detector means (4) for emission and detection of a radiation given, said source and detector means (4) being designed to rotate about the area of analysis (5) for acquiring radiographies of the object; actuator means (21-23) for moving the supporting means (3) with respect to the source and detector means (4) along three axes of displacement (18-20); processing means (13), designed to process the radiographies so as to reconstruct images of the object and to supply displacement instructions for the actuator means (21-23); and control means (12) for controlling the actuator means (21-23) according to said displacement instructions; the processing means (13) comprising display means (14) for displaying the images of the object and pointer means (15, 16) for moving a pointer (24) on the images displayed; the processing means (13) being configured for implementing the method for positioning an object according to any one of Claims 1 to 6.

8. The computed tomography scanner according to Claim 7, in which said pointer means (15, 16) comprise a keyboard (15).

9. The computed tomography scanner according to Claim 7 or Claim 8, in which said pointer means (15, 16) comprise a mouse (16).

10. A computed tomography scanner for use in dentistry **characterized in that** it is built according to any one of Claim 7 to 9.
